(19) Europäisches Patentamt

European Patent Office

Office européen des brevets

(11) **EP 1 222 154 B1**

(12) **FASCICULE DE BREVET EUROPEEN**

(45) Date de publication et mention
de la délivrance du brevet:
**26.05.2004 Bulletin 2004/22**

(51) Int Cl.[7]: **C07C 17/278**, C07C 17/275,
C07C 19/01, C07C 19/08,
C07C 17/20

(21) Numéro de dépôt: **00967818.6**

(22) Date de dépôt: **26.09.2000**

(86) Numéro de dépôt international:
**PCT/EP2000/009490**

(87) Numéro de publication internationale:
**WO 2001/025176 (12.04.2001 Gazette 2001/15)**

(54) **PROCEDE DE PREPARATION D'HYDROCARBURES HALOGENES**

VERFAHREN ZUR HERSTELLUNG VON HALOGENIERTEN KOHLENWASSERSTOFFEN

METHOD FOR PREPARING HALOGENATED HYDROCARBONS

(84) Etats contractants désignés:
**AT BE CH CY DE DK ES FI FR GB GR IE IT LI LU MC NL PT SE**

(30) Priorité: **06.10.1999 EP 99203270**

(43) Date de publication de la demande:
**17.07.2002 Bulletin 2002/29**

(73) Titulaire: **SOLVAY (Société Anonyme)**
**1050 Bruxelles (BE)**

(72) Inventeurs:
• **MATHIEU, Véronique**
**B-1300 Wavre (BE)**

• **ANCIAUX, Charles-Marie**
**B-1180 Bruxelles (BE)**

(74) Mandataire: **Jacques, Philippe et al**
**Solvay S.A.**
**Département de la Propriété Industrielle,**
**Rue de Ransbeek, 310**
**1120 Bruxelles (BE)**

(56) Documents cités:
**EP-A- 0 131 561        EP-A- 0 787 707**
**WO-A-98/50330        FR-A- 2 120 337**
**GB-A- 1 146 463**

**Description**

La présente invention concerne un procédé de préparation d'hydrocarbures halogénés comprenant au moins 3 atomes de carbone par réaction catalytique entre un haloalcane et une oléfine.

L'addition d'un haloalcane sur une oléfine est une réaction bien connue. Cependant, il est parfois difficile de contrôler la réaction de telle sorte qu'une seule molécule d'oléfine s'additionne à une molécule d'haloalcane (formation d'un produit d'addition ou adduit 1:1).

La demande de brevet WO 97/07083 décrit un procédé de préparation d'hydrocarbures halogénés sous l'action catalytique du chlorure cuivreux en présence de t.butylamine comme cocatalyseur. Dans un tel procédé, le rendement en produit de télomérisation est toutefois assez faible.

La demande de brevet EP-A-787707 décrit un procédé de préparation de 1,1,1,3,3-pentachlorobutane sous l'action catalytique du chlorure cuivreux en présence de cocatalyseurs de type amine. Le rendement et la sélectivité en produit de télomérisation ne sont toutefois pas excellents.

La présente invention vise à fournir un procédé de télomérisation permettant de préparer des hydrocarbures halogénés comprenant au moins 3 atomes de carbone par réaction catalytique entre un haloalcane et une oléfine avec des rendements et sélectivités améliorés.

En conséquence, l'invention concerne un procédé de préparation d'hydrocarbures halogénés comprenant au moins 3 atomes de carbone dans lequel on fait réagir un haloalcane et une oléfine en présence d'un catalyseur dans un milieu réactionnel, procédé dans lequel le milieu réactionnel est essentiellement exempt d'eau.

Il a en effet été trouvé, de manière surprenante, que lorsqu'on fait réagir un haloalcane et une oléfine en présence d'un catalyseur dans un milieu réactionnel essentiellement exempt d'eau, on augmente à la fois la sélectivité en monoadduits désirables d'haloalcane et d'oléfine et la conversion de l'oléfine, ce qui résulte en une productivité élevée en hydrocarbures halogénés désirables comprenant au moins 3 atomes de carbone.

Dans le procédé selon l'invention, le milieu réactionnel comprend au moins le catalyseur, l'haloalcane et l'oléfine. Le milieu réactionnel peut comprendre en outre, par exemple, un cocatalyseur et/ou un solvant, de préférence un cocatalyseur.

Le milieu réactionnel contient généralement au plus 1300 mg/kg d'eau. Souvent, le milieu réactionnel contient au plus 1000 mg/kg d'eau. Un milieu réactionnel contenant au plus 700 mg/kg d'eau convient bien. De préférence le milieu réactionnel contient au plus 400 mg/kg d'eau. Un milieu réactionnel contenant au plus 300 mg/kg d'eau est tout particulièrement préféré. Le procédé peut bien sûr être mis en oeuvre dans un milieu réactionnel totalement anhydre. Il s'est toutefois avéré que la présence de traces d'eau en faibles quantités n'est pas gênante. Typiquement, le procédé selon l'invention est mis en oeuvre avec un milieu réactionnel contenant plus de 20 mg/kg d'eau, voire plus de 50 mg/kg d'eau.

La teneur en eau du milieu réactionnel peut être contrôlée, par exemple, en éliminant des traces d'eau du réacteur utilisé. On peut, par exemple, préalablement à l'introduction du milieu réactionnel, chauffer le réacteur et/ou effectuer des purges du réacteur avec un gaz sec. De plus, on peut réduire la teneur en eau dans les constituants du milieu réactionnel, par exemple, dans le catalyseur, l'haloalcane et l'oléfine, et éventuellement dans le cocatalyseur et/ou le solvant. Des opérations utilisables pour réduire la teneur en eau dans les constituants du milieu réactionnel sont, par exemple, une opération de séchage, tel que par exemple une adsorption sur un adsorbant solide ou une opération de distillation.

Dans les installations industrielles, il est désirable de recycler certains composés présents dans le milieu réactionnel en fin de réaction vers l'étape dans laquelle on fait réagir l'oléfine et l'haloalcane en présence d'un catalyseur.

Le procédé selon l'invention concerne donc un procédé dans lequel

(a) on fait réagir un haloalcane et une oléfine en présence d'un catalyseur dans un milieu réactionnel essentiellement exempt d'eau ;
(b) on soumet à un traitement de réduction de la teneur en eau au moins une fraction du mélange réactionnel issu de l'étape (a) ;
(c) on recycle au moins une partie de la fraction traitée à l'étape (b) vers l'étape (a).

Ce procédé est particulièrement utile lorsqu'on soumet au moins une fraction d'un mélange réactionnel issu de l'étape dans laquelle on fait réagir l'oléfine et l'haloalcane en présence d'un catalyseur contenant des réactifs non consommés à au moins un traitement avec un milieu aqueux. Le cas échéant le traitement avec le milieu aqueux a notamment pour but d'arrêter la réaction entre l'oléfine et l'haloalcane et de séparer le catalyseur et éventuellement le cocatalyseur des autres constituants du milieu réactionnel. Dans une variante préférée, on isole du cocatalyseur du milieu aqueux par une opération de séparation telle que par exemple un stripping ou une distillation et, si nécessaire, on soumet le cocatalyseur séparé à un traitement supplémentaire de réduction de la teneur en eau préalablement au recyclage vers la réaction entre l'oléfine et l'haloalcane.

La fraction soumise au traitement de réduction de la teneur en eau peut contenir des réactifs non consommés (oléfine et/ou haloalcane), du catalyseur, du cocatalyseur, du solvant ou des mélanges de ces composés. Dans une variante préférée, elle contient de l'oléfine. Dans une autre variante préférée, elle contient du cocatalyseur. Dans encore une autre variante, elle contient de l'haloalcane.

Le procédé selon l'invention peut être un procédé continu ou discontinu. Dans un procédé discontinu on préfère effectuer une addition progressive au milieu réactionnel en cours de réaction d'au moins une partie d'au moins un réactif sélectionné parmi l'oléfine, et l'haloalcane et éventuellement le cocatalyseur et/ou le solvant.

Dans le procédé selon l'invention, le catalyseur peut être choisi parmi les dérivés de métal connus pour catalyser la réaction d'un haloalcane avec une oléfine. Citons, par exemple, les sels et les composés organiques des métaux des groupes VIA, VIIA, VIII et IB du système périodique des éléments. Parmi ces métaux, le nickel, le fer et le cuivre donnent de bons résultats. Des composés de cuivre conviennent bien à titre de catalyseur. Les sels de cuivre et les composés organiques de cuivre sont particulièrement préférés à titre de catalyseur. De tels catalyseurs, sont décrits, par exemple, dans les demandes de brevet WO-A-98/50329 et WO-A-98/50330 dont le contenu à ce sujet est incorporé par référence. Le chlorure de cuivre (I), le chlorure de cuivre (II) ou l'acétylacétonate de cuivre (II) donnent de bons résultats. Des sels de cuivre hydratés peuvent être mis en oeuvre pour autant qu'ils n'introduisent pas dans le milieu réactionnel une teneur en eau supérieure à la teneur spécifiée plus haut.

Toutefois, on préfère mettre en oeuvre des sels de cuivre anhydres. De manière particulièrement préférée on met en oeuvre un sel de cuivre (II) anhydre. Convient particulièrement bien un sel de cuivre (II) anhydre choisi parmi les halogénures ou l'acétate de cuivre(II), en particulier le chlorure de cuivre(II) anhydre.

Dans le procédé selon l'invention, on réalise la réaction de préférence en présence d'un cocatalyseur sélectionné, par exemple, dans le groupe constitué des amines et des oxydes de trialkylphosphine.

On préfère utiliser une amine comme cocatalyseur, en particulier une amine primaire. Des amines utilisables sont notamment la t.butylamine et les amines tert-alkyles PRIMENE® 81-R et JM-T commercialisées par Rohm and Haas Company. La t.butylamine, les amines PRIMENE® 81-R et PRIMENE®JM-T sont tout particulièrement préférées. L'amine PRIMENE® 81-R est un mélange d'amines primaires tert.-alkyle dont le nombre d'atomes de carbone est de 12 à 14. L'amine PRIMENE® JM-T est un mélange d'amines primaires tert.-alkyle dont le nombre d'atomes de carbone est de 18 à 22.

Parmi les oxydes de trialkylphosphine utilisables comme cocatalyseur, on peut citer notamment les composés de formule (R1R2R3)PO, dans laquelle R1, R2 et R3 représentent des groupements alkyles en C3-C10, identiques ou différents, de préférence linéaires. On retient en particulier l'oxyde de tri-(n-butyl)phosphine, l'oxyde de tri-(n-hexyl) phosphine, l'oxyde de tri-(n-octyl)phosphine, l'oxyde de n-octyl-di-(n-hexyl)-phosphine et l'oxyde de n-hexyl-di-(n-octyl)-phosphine et leurs mélanges.

Le système catalyseur-cocatalyseur préféré dans le procédé selon l'invention est le système constitué d'un composé de cuivre et d'une amine primaire dont l'atome de carbone voisin du groupe $NH_2$ est un atome de carbone quaternaire, c'est-à-dire dépourvu d'atome d'hydrogène. Sont particulièrement préférés, les systèmes catalyseur-cocatalyseur formés par l'acétylacétonate de cuivre (II) et la t.butylamine ou par le chlorure de cuivre (II) et la t.butylamine.

Un système catalyseur-cocatalyseur tout particulièrement préféré est le système formé par l'acétylacétonate de cuivre (II) et la PRIMENE®JM-T.

Dans le procédé selon l'invention, la réaction est de préférence réalisée en l'absence de solvant. Toutefois, la réaction peut également être réalisée en présence d'un solvant. Tout solvant dans lequel les réactifs forment le produit recherché avec un rendement satisfaisant peut être utilisé.

Les haloalcanes engagés dans le procédé selon la présente invention sont en général des composés organiques saturés. Ils possèdent de préférence de un à trois atomes de carbone. Souvent ils contiennent au moins 2 atomes de chlore. Des haloalcanes dans lesquels au moins 2 atomes de chlore sont liés au même atome de carbone donnent des bons résultats. Les haloalcanes comprenant un groupement dichlorométhyle ou trichlorométhyle sont préférés. A titre d'exemples d'haloalcanes selon la présente invention, on peut citer le dichlorométhane, le chloroforme, le tétrachlorure de carbone, le 1,1,1-trichloroéthane. Le tétrachlorure de carbone est tout particulièrement préféré. Les haloalcanes peuvent également comprendre d'autres substituants tels que d'autres atomes d'halogène, des groupes alkyle ou halogénoalkyle. Toutefois, les haloalcanes contenant seulement du chlore à titre d'halogène sont préférés.

L'oléfine mise en oeuvre dans le procédé selon la présente invention est en général un éthylène, un propylène ou un butène, éventuellement substitué par un substituant. Les substituants sont choisis, par exemple, parmi les atomes d'halogène, les groupes alkyle ou halogénoalkyle, des groupes nitrile (CN) ou acide carboxylique (COOH). Les oléfines halogénées conviennent particulièrement bien. Parmi les oléfines halogénées, les oléfines chlorées donnent de bons résultats. Des oléfines chlorées utilisables dans le procédé selon l'invention répondent généralement à la formule générale

R1ClC=CR2R3

(I)

dans laquelle R1, R2, et R3 représentent indépendamment un atome H ou Cl, un groupement alkyle ou alcényle, linéaire, cyclique ou ramifié, éventuellement substitué, ou un groupement aryle ou héteroaryle, éventuellement substitué.

A titre d'exemples non limitatifs d'oléfines chlorées on peut citer le chlorure de vinyle, le chlorure de vinylidène, le trichloréthylène, les divers isomères des chloropropènes comme le 1-chloroprop-1-ène, le 2-chloroprop-1-ène et le 3-chloroprop-1-ène. D'excellents résultats peuvent être obtenus avec le chlorure de vinyle et le 2-chloroprop-1-ène.

Les hydrocarbures halogénés obtenus selon le procédé de la présente invention appartiennent, en général, à la famille des alcanes chlorés comprenant au moins trois atomes de carbone. Il s'agit souvent des chloropropanes, des chlorobutanes ou des chloropentanes. Les atomes de carbone desdits chloropropanes, chlorobutanes et chloropentanes peuvent également être substitués par d'autres groupes fonctionnels, comme, par exemple, des groupes alkyle ou aryle, des groupes nitrile (CN) ou acide carboxylique (COOH). Souvent les hydrocarbures halogénés contiennent uniquement du chlore à titre d'halogène. Les chloropropanes et les chlorobutanes non substitués par d'autres groupes fonctionnels sont préférés.

De préférence, les hydrocarbures halogénés obtenus selon le procédé de la présente invention répondent à la formule générale $CnH_{(2n+2)-p}Cl_p$ dans laquelle n est un nombre entier et possède les valeurs 3 ou 4, p est un nombre entier qui possède les valeurs 3 à 7. Des exemples de composés obtenus selon le procédé de la présente invention sont le 1,1,1,3,3-pentachloropropane, le 1,1,2,3,3-pentachloropropane, le 1,1,1,3,3-pentachlorobutane, le 1,1,1,3-tétrachloropropane, le 1,1,3,3-tétrachlorobutane, le 1,1,1,3,3,3-hexachlorororopropane et le 1,1-dichloro-2-trichlorométhylpropane. Parmi ces composés, le 1,1,1,3,3-pentachloropropane, le 1,1,1,3,3-pentachlorobutane et le 1,1,1,3,3,3-hexachlorororopropane sont préférés. Le 1,1,1,3,3-pentachlorobutane et le 1,1,1,3,3-pentachloropropane sont tout particulièrement préférés.

Le milieu réactionnel est maintenu à une température et une pression suffisante pour permettre la réaction catalytique de l'haloalcane avec l'oléfine. Les conditions de réaction préférées dans le procédé selon l'invention quant à la température, pression, durée et le rapport entre les constituants du milieu réactionnel etc. sont décrites dans la demande de brevet WO-A-98/50330 dont le contenu est incorporé par référence.

Les hydrocarbures halogénés obtenus selon le procédé de l'invention sont des précurseurs des analogues fluorés correspondants, lesquels peuvent être facilement obtenus par traitement avec du fluorure d'hydrogène, de préférence anhydre, éventuellement en présence d'un catalyseur de fluoration tel que par exemple un sel d'antimoine, un sel de titane, un sel de tantale ou un sel d'étain. Les halogénures sont préférés à titre de sel desdits métaux. D'autres catalyseurs de fluoration utilisables sont choisis parmi les composés, de préférence les oxydes, de chrome, d'alumine et de zirconium. Des exemples spécifiques d'hydrocarbures fluorés répondent à la formule $C_nH_{(2n+2)-p}F_p$ dans laquelle n est un nombre entier et possède les valeurs 3 ou 4 et p est un nombre entier qui possède les valeurs 3 à 7. Des hydrocarbures fluorés préférés sont choisis parmi le 1,1,1,3,3-pentafluorpropane, le 1,1,1,3,3,3-hexafluorpropane et le 1,1,1,3,3,-pentafluorbutane.

L'invention concerne dès lors aussi une méthode d'obtention d'un hydrocarbure fluoré comprenant (a) la synthèse d'un hydrocarbure halogéné selon le procédé selon l'invention et (b) un traitement de l'hydrocarbure halogéné issu de (a) avec du fluorure d'hydrogène tel que décrit plus haut. Les exemples ci-après entendent illustrer l'invention, sans toutefois la limiter.

## Exemples 1-4

On a préparé du 1,1,1,3,3-pentachlorobutane par réaction entre du 2-chloroprop-1-ène (2-CPe) et du tétrachlorure de carbone en présence de chlorure cuivrique et de t-butylamine (tBu) en présence de quantités variables d'eau. Pour ce faire, on a introduit les réactifs (2-CPe, $CCl_4$), le catalyseur ($CuCl_2$) et l'amine (tBu) dans un flacon en verre de type pénicilline dans des rapports molaires 2-CPe/$CCl_4$/$CuCl_2$/tBu de 1/2/0,002/0,1 et on a ajouté une certaine quantité d'eau. Le flacon a ensuite été fermé hermétiquement et placé dans un bain d'huile chauffé à 80 °C. L'agitation a été assurée par un barreau magnétique. Après 0,5 h de réaction, un échantillon de liquide a été prélevé à la seringue et dosé par une méthode chromatographique pour déterminer le taux de conversion de l'oléfine. Les résultats sont rassemblés dans le tableau ci-après, ainsi que la sélectivité en 1,1,1,3,3-pentachlorobutane obtenue après 3 heures de réaction.

Tableau

| Exemple | [H$_2$O], mg/kg | Conversion de 2-chloroprop-1-ène, % | Sélectivité en HCC-360, % |
|---------|-----------------|--------------------------------------|----------------------------|
| 1 | 249 | 83 | 96,4 |
| 2 | 639 | 60 | 95,6 |
| 3 | 679 | 30 | 91,2 |
| 4 | 1349 | 5 | 73,0 |

Exemple 5

On a préparé du 1,1,1,3,3-pentachlorobutane par réaction, en milieu substantiellement anhydre, entre du 2-chloroprop-1-ène (2-CPe) et du tétrachlorure de carbone en présence d'acétylacétonate de cuivre, et de l'amine PRIMENE® JM-T (Rohm&Haas). Pour ce faire, on a introduit les réactifs (2-CPe, CCl$_4$), le catalyseur (Cu(acac)$_2$) et l'amine (JM-T) dans un réacteur dans des rapports molaires 2-CPe/CCl$_4$/Cu(acac)$_2$/JM-T de 1/2/0,001/0,22. Le réacteur a ensuite été chauffé à 90°C. Après 2 h de réaction, un échantillon de liquide a été prélevé à la seringue et dosé par une méthode chromatographique pour déterminer la composition du milieu réactionnel. Le taux de conversion du 2-CPe a été de 100% et la sélectivité en 1,1,1,3,3-pentachlorobutane a été de 95%.

**Revendications**

1. Procédé de préparation d'hydrocarbures halogénés comprenant au moins 3 atomes de carbone selon lequel :

   (a) on fait réagir un haloalcane et une oléfine en présence d'un catalyseur dans un milieu réactionnel essentiellement exempt d'eau;
   (b) on soumet à un traitement de réduction de la teneur en eau au moins une fraction du mélange réactionnel issu de l'étape (a);
   (c) on recycle au moins une partie de la fraction traitée à l'étape (b) vers l'étape (a).

2. Procédé selon la revendication 1, dans lequel le milieu réactionnel contient au plus 1000 mg/kg d'eau.

3. Procédé selon la revendication 1, dans lequel le milieu réactionnel contient an plus 700 mg/kg d'eau.

4. Procédé selon la revendication 1, dans lequel le milieu réactionnel contient au plus 400 mg/kg d'eau.

5. Procédé selon l'une quelconque des revendications 1 à 4, dans lequel on fait réagir l'haloalcane et l'oléfine en outre en présence d'un cocatalyseur.

6. Procédé selon l'une quelconque des revendications 1 à 5, dans lequel le catalyseur est un composé de cuivre.

7. Procédé selon la revendication 5 ou 6, dans lequel le cocatalyseur est une amine.

8. Procédé selon l'une quelconque des revendications 1 à 7, dans lequel l'oléfine est une oléfine halogénée.

9. Procédé selon la revendication 8, dans lequel l'oléfine halogénée est une oléfine chlorée répondant à la formule générale R1ClC=CR2R3 dans laquelle R1, R2 et R3 représentent indépendamment : H, Cl, alkyle ou alcényle linéaire, cyclique ou ramifié, éventuellement substitué ; aryle ou héteroaryle éventuellement substitué.

10. Procédé selon l'une quelconque des revendications 1 à 9, dans lequel l'hydrocarbure halogéné préparé est choisi parmi le 1,1,1,3,3-pentachloropropane, le 1,1,2,3,3-pentachloropropane, le 1,1,1,3,3-pentachlorobutane, le 1,1,1,3-tétrachloropropane, le 1,1,3,3-tétrachlorobutane, le 1,1,1,3,3,3-hexachlororopropane et le 1,1-dichloro-2-trichlorométhylpropane.

11. Procédé selon l'une quelconque des revendications 1 à 10, dans lequel la fraction de mélange réactionnel issue de l'étape (a) contenant des réactifs non consommés est soumise, préalablement à l'étape (b), à au moins un traitement avec un milieu aqueux.

**12.** Méthode d'obtention d'un hydrocarbure fluoré comprenant :

(a) la synthèse d'un hydrocarbure halogéné selon le procédé selon l'une quelconque des revendications 1 à 11
(b) un traitement de l'hydrocarbure halogéné obtenu en (a) avec du fluorure d'hydrogène.

**Patentansprüche**

**1.** Verfahren zur Herstellung von halogenierten Kohlenwasserstoffen, die wenigstens 3 Kohlenstoffatome umfassen, wonach man

(a) ein Halogenalkan und ein Olefin in Gegenwart eines Katalysators in einem im wesentlichen wasserfreien Reaktionsmilieu umsetzt;

(b) wenigstens eine Fraktion des aus der Stufe (a) erhaltenen Reaktionsgemisches einer Behandlung zur Verringerung des Wassergehaltes unterzieht;

(c) wenigstens einen Teil der in Stufe (b) behandelten Fraktion zur Stufe (a) recycliert.

**2.** Verfahren nach Anspruch 1, worin das Reaktionsmilieu höchstens 1000 mg/kg Wasser enthält.

**3.** Verfahren nach Anspruch 1, worin das Reaktionsmilieu höchstens 700 mg/kg Wasser enthält.

**4.** Verfahren nach Anspruch 1, worin das Reaktionsmilieu höchstens 400 mg/kg Wasser enthält.

**5.** Verfahren nach einem der Ansprüche 1 bis 4, worin man das Halogenalkan mit dem Olefin überdies in Gegenwart eines Co-Katalysators umsetzt.

**6.** Verfahren nach einem der Ansprüche 1 bis 5, worin der Katalysator eine Kupferverbindung ist.

**7.** Verfahren nach Anspruch 5 oder 6, worin der Co-Katalysator ein Amin ist.

**8.** Verfahren nach einem der Ansprüche 1 bis 7, worin das Olefin ein halogeniertes Olefin ist.

**9.** Verfahren nach Anspruch 8, worin das halogenierte Olefin ein chloriertes Olefin entsprechend der allgemeinen Formel $R_1ClC=CR_2R_3$ ist, worin $R_1$, $R_2$ und $R_3$ unabhängig für H, Cl, gerades, cyclisches oder verzweigtes, gegebenenfalls substituiertes Alkyl oder Alkenyl oder gegebenenfalls substituiertes Aryl oder Heteroaryl stehen.

**10.** Verfahren nach einem der Ansprüche 1 bis 9, worin der hergestellte halogenierte Kohlenwasserstoff unter 1,1,1,3,3-Pentachlorpropan, 1,1,2,3,3-Pentachlorpropan, 1,1,1,3,3-Pentachlorbutan, 1,1,1,3-Tetrachlorpropan, 1,1,3,3-Tetra-chlorbutan, 1,1,1,3,3,3-Hexachlorpropan und 1,1-Dichlor-2-trichlormethylpropan gewählt wird.

**11.** Verfahren nach einem der Ansprüche 1 bis 10, worin die aus der Stufe (a) kommende Fraktion des Reaktionsgemisches, die nicht verbrauchte Reaktanten enthält, vor der Stufe (b) wenigstens einer Behandlung mit einem wässerigen Milieu unterworfen wird.

**12.** Verfahren zur Gewinnung eines fluorierten Kohlenwasserstoffes, umfassend

(a) die Synthese eines halogenierten Kohlenwasserstoffes nach dem Verfahren gemäß einem der Ansprüche 1 bis 11 und

(b) eine Behandlung des in (a) erhaltenen halogenierten Kohlenwasserstoffes mit Fluorwasserstoff.

**Claims**

**1.** Process for preparing halohydrocarbons containing at least 3 carbon atoms, in which

(a) a haloalkane and an olefin are reacted in the presence of a catalyst in a reaction medium, which is essentially free of water;

(b) at least a fraction of a reaction mixture obtained from the step is subjected to a treatment to reduce the water content;

(c) at least some of the treated fraction is recycled into the step in which the olefin and the haloalkane are reacted.

2. Process according to Claim 1, in which the reaction medium contains not more than 1000 mg/kg of water.

3. Process according to Claim 1, in which the reaction medium contains not more than 700 mg/kg of water.

4. Process according to Claim 1, in which the reaction medium contains not more than 400 mg/kg of water.

5. Process according to any one of Claims 1 to 4, in which the haloalkane and the olefin are also reacted in the presence of a co-catalyst.

6. Process according to any one of Claims 1 to 5, in which the catalyst is a copper compound.

7. Process according to Claim 5 or 6, in which the co-catalyst is an amine.

8. Process according to any one of Claims 1 to 7, in which the olefin is a haloolefin.

9. Process according to Claim 8, in which the haloolefin is a chloroolefin corresponding to the general formula $R_1ClC=CR_2R_3$ in which $R_1$, $R_2$ and $R_3$ independently represent: H, Cl, linear, cyclic or branched, optionally substituted alkyl or alkenyl; optionally substituted aryl or heteroaryl.

10. Process according to any one of Claims 1 to 9, in which the halohydrocarbon prepared is chosen from 1,1,1,3,3-pentachloropropane, 1,1,2,3,3-pentachloropropane, 1,1,1,3,3-pentachlorobutane, 1,1,1,3-tetrachloropropane, 1,1,3,3-tetrachlorobutane, 1,1,1,3,3,3hexachloropropane and 1,1-dichloro-2-trichloromethylpropane.

11. Process according to anyone of claims 1 to 10, in which the fraction of the reaction mixture obtained from step (a) containing unconsumed agents is subjected before step (b) to at least one treatement with an aqueous medium.

12. Method for obtaining a fluorohydrocarbon, comprising

(a) the synthesis of a halohydrocarbon according to the process according to any one of Claims 1 to 11,

(b) a treatment of the halohydrocarbon obtained in (a) with hydrogen fluoride.